(19) Europäisches Patentamt

European Patent Office

Office européen des brevets

(11) **EP 4 025 294 B1**

(12) **EUROPEAN PATENT SPECIFICATION**

(45) Date of publication and mention
of the grant of the patent:
**18.12.2024 Bulletin 2024/51**

(21) Application number: **20793042.1**

(22) Date of filing: **04.09.2020**

(51) International Patent Classification (IPC):
**A61N 1/32** *(2006.01)*   **A61N 1/05** *(2006.01)*
**A61N 1/36** *(2006.01)*   **A61N 1/362** *(2006.01)*

(52) Cooperative Patent Classification (CPC):
**A61N 1/323; A61N 1/0529; A61N 1/36014;**
A61N 1/0551; A61N 1/36034; A61N 1/3625

(86) International application number:
**PCT/GB2020/052134**

(87) International publication number:
**WO 2021/044168 (11.03.2021 Gazette 2021/10)**

(54) **APPARATUS AND METHOD**

VORRICHTUNG UND VERFAHREN

APPAREIL ET MÉTHODE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.09.2019   GB 201912887**

(43) Date of publication of application:
**13.07.2022   Bulletin 2022/28**

(73) Proprietor: **IMPERIAL COLLEGE INNOVATIONS
LIMITED**
**London SW7 2AZ (GB)**

(72) Inventors:
  • **DZIALECKA, Patrycja**
    **London SW7 2AZ (GB)**

  • **GROSSMAN, Nir**
    **London SW7 2AZ (GB)**

(74) Representative: **Leach, Sean Adam et al**
**Mathys & Squire**
**The Shard**
**32 London Bridge Street**
**London SE1 9SG (GB)**

(56) References cited:
**EP-A1- 0 002 811        WO-A1-2016/057855
WO-A1-2017/023914    WO-A2-2010/010567
US-A- 5 324 317**

## Description

## Technical Field

The present disclosure relates generally to stimulation of biological tissue comprising electrically excitable cells, and more particularly to methods and apparatus for the control of electrical signals for use in the stimulation of such tissue by interferential stimulation.

## Background

Some cells in biological tissue have the ability to be electrically excited, by the application of electric field, to cause the cell to generate action potentials. Neurons, muscle cells (skeletal, cardiac, and smooth), and some endocrine cells (e.g., insulin-releasing pancreatic β cells) are some examples of electrically excitable cells.

One example of use of this phenomenon is in so-called deep brain stimulation. Physical means of brain stimulation, sometimes referred to as 'neuromodulation', offer a tenable, non-pharmacological means to probe and treat brain disorders through direct control of circuit activity. Implanted electrodes for deep brain stimulation (DBS) are CE and FDA-approved therapies for severe movement disorders, such as Parkinson disease, and affective disorders, such as obsessive-compulsive disorder. However, such methods are necessarily invasive and it would be preferable to provide a method of brain stimulation which is non-invasive.

Precisely directed stimulation of arbitrary regions of biological tissue, such as brain tissue, may be achieved by interferential stimulation. Electrodes positioned around the tissue produce a number of time-varying electric fields which interfere with each other (e.g. by superposition) to produce a combined time-varying electric field in a region in which the electric fields overlap. Provided the frequency of the combined time-varying field is in the correct frequency band, and the amplitude is sufficient, the combined field may excite excitable cells in the tissue - e.g. evoke action potentials in those cells. In the case of brain tissue, this may recruit effective neural firing.

Regions of the brain which may be targeted are not limited to the area immediately beneath the electrodes (e.g. regions on an exterior surface of the brain), but may be any superficial, mid-depth or deep brain structure. For example, in some scenarios, interferential stimulation is precisely targeted at a deep brain structure, such as the thalamus, hypothalamus, amygdala, or hippocampus. In other scenarios, interferential stimulation is precisely targeted on a superficial or mid-depth region of the cortex. In yet other scenarios, interferential stimulation is precisely targeted on both a superficial brain structure and deep brain structure simultaneously.

WO2016/057855 describes methods and apparatus for stimulation of biological tissue. In particular, interferential stimulation is precisely directed to arbitrary regions in a brain. The target region is not limited to the area immediately beneath the electrodes, but may be any superficial, mid-depth or deep brain structure. US 5324317 A relates to an interferential generator for treating a living body with low frequency therapeutic current at a selected point. EP 0002811 A1 describes an apparatus for interference current therapy with a common oscillator for both the circuits present.

The present disclosure provides an improved method and apparatus of interferential stimulation of a brain.

## Summary

Aspects of the invention are set out in the independent claims. Optional features are set out in the dependent claims.

An aspect provides an apparatus for providing electric field in biological tissue for causing stimulation of that tissue. For example, stimulation may comprise providing electric field in the tissue of sufficient amplitude to evoke action potentials in electrically excitable cells in the tissue. This may modulate activity of the tissue.

Such an apparatus may comprise: a first electrical signal provider for providing a first alternating electric field in the biological tissue, the first alternating electric field having a first frequency; a second electrical signal provider for providing a second alternating electric field in the biological tissue the second alternating electric field having a second frequency. The first alternating electric field and the second alternating electric field provide a combined field in the biological tissue, for example such as would be provided by the superposition of the two alternating electric fields. The apparatus comprises a controller configured to provide variations of at least one of the first frequency and the second frequency.

The present disclosure also provides a method of stimulating biological tissue, the method comprising: controlling a first alternating current at a first location thereby to provide a first alternating electric field in the biological tissue, the first alternating electric field having a first frequency; providing a second alternating current at a second location thereby to provide a second alternating electric field in the biological tissue the second alternating electric field having a second frequency; wherein the first electric field and the second electric field combine to provide a combined field in the biological tissue at a third location, and the method comprises: varying at least one of the first frequency and the second frequency so that the combined field provides a pulsed interferential stimulation signal. At least one of the first location and the second location may be in the biological tissue which is to be stimulated. It will be appreciated in the context of the present disclosure that although the apparatus and methods described herein may be used in or on the living human or animal body, it may also be useful in biological tissue *ex vivo*. It may also be used in instances wherein the method is not a method of treatment of the living human or animal body by surgery or therapy.

The variations in frequency may be controlled to provide an interval in which there is a frequency difference between the two alternating electric fields. During this interval the difference in frequency causes amplitude modulation (e.g. beats) in the combined field. The signals may be controlled so that the resultant modulation depth is greater than a so-called "threshold amplitude". This may be the amplitude necessary for a single electric field pulse to evoke an action potential in the electrically excitable cells. During a preceding and/or subsequent interval the frequencies may match e.g. be the same.

This is one way in which variations in frequency may provide a pulsed stimulation signal in the combined field. This can provide focal (e.g. localised) stimulation of the biological tissue in a region which is remote from the currents/electrodes which generate the field whilst reducing unwanted activation of tissue due to the direct effect of those currents/electrodes themselves. Embodiments may provide a series of first intervals during which the first frequency matches the second frequency. These first intervals may be interleaved with a series of second intervals during which the frequency difference between the two electric fields provides amplitude modulation of the combined field. The interleaving of these periods can provide a series of pulses of the interferential stimulation signal. The frequency difference, $\Delta f$, during the second intervals may comprise a frequency in the natural band of the electrically excitable cells. This frequency difference may provide a beat period, $1/\Delta f$. The second intervals may have a duration which is an integer multiple of the beat period.

The length of the first intervals (e.g. the inter-pulse interval) may also be chosen according to the type of biological tissue and/or the desired type of activation of that tissue. For example, the pulse frequency (reciprocal of inter-pulse interval) may be chosen to be within the natural band of the tissue and/or cell type to be stimulated. It will be appreciated that these first intervals may provide a time in which the interferential stimulation signal is of sub-threshold amplitude, e.g. not sufficient to evoke an action potential in the electrically excitable cells. The match of frequencies during these first intervals need not require the frequencies to be equal provided that any beats in the combined field due to mismatch have a frequency which is outside the natural band of the electrically excitable cells.

Temporal interference stimulation 3D focal and steerable stimulation of biological cells. In the context of stimulating excitable cells (e.g., neurons or cardiomyocytes), the pulsation of the TI stimulation can make it more efficient since it a) can lower the threshold of evoking action potential at the focus of the TI due to a faster depolarization rate of the membrane and b) can better synchronize a large number of cells, e.g., neural network, at the focus of the TI since the stimulating pattern (i.e., the change in the envelope amplitude of the superimposed kHz fields) is more localized in time. In addition, the pulsation of the TI stimulation enable it to be applied to already efficient neuromodulation protocols that are based on pulses of electric fields, e.g., electroconvulsive therapy and theta-burst stimulation, with the benefit of 3D focality.

The apparatus and methods described herein may be applied to a variety of types of cells and tissues. For example, the tissue may comprise excitable cells such as neurons or cardiomyocytes. Examples of such tissues include brain tissue, cardiac tissue, and nerve tissue such as that found in the spinal cord. Accordingly, the variations in frequency may be chosen to provide a frequency difference, $\Delta f$, during the second ("on") intervals to provide activation of the tissue type concerned. The frequency difference may thus be chosen so that the beat frequency is greater than the minimum frequency of the natural band of the cell. For example, in nerve tissue and brain tissue, the frequency difference may be chosen to provide neuro-stimulation. The amplitude (e.g. the amplitude of oscillation) of the first alternating electric field and the second alternating electric field may be held constant. This need not be absolutely constant, merely stable enough that any variation in this amplitude is less than the threshold amplitude necessary for a single electric field pulse to evoke an action potential in the given cell. Larger variations may also be possible, e.g. long term drifts, but typically these will provide signal contributions outside the natural band of the electrically excitable cells.

Typically the first frequency and the second frequency are both greater than a minimum frequency selected to avoid stimulation of the biological tissue. This minimum may be chosen according to the type of electrically excitable cells. For example, typically both frequencies are greater than any frequency within the natural band of the electrically excitable cells. For example, the minimum frequency may be at least 500Hz, for example at least 800 Hz, for example at least 1KHz. Such minimum frequencies may be relevant to brain or spinal cord tissue, but may also be relevant to other tissues

The first electrical signal provider may comprise a first current source for providing electrical current between a first two electrodes, thereby to provide the first electric field, and the second electrical signal provider may comprise a second current source for providing electrical current between two electrodes, thereby to provide the second electric field. These electrodes may comprise implantable stimulating electrodes suitable for implantation in biological tissue, such as brain or cardiac tissue. The electrodes may also comprise electrodes suitable to be secured to some other structure adjacent the tissue to be stimulated. For example the electrodes may be configured to be secured to the skin (e.g. the scalp), for example outside the human or animal body.

The pulses may have a duration of at least 5ms, and may have a duration of less than 150ms, for example less than 25ms. The pulses have a duration of between 5 ms and 25 ms

The amplitude of the first alternating electric field and the second alternating electric field may be kept constant while the frequency is varied. The frequency of one of the two alternating signals may be kept constant while the frequency of the other is varied to provide the frequency difference which provides the amplitude modulation.

The frequency modulation may be controlled to provide a train of pulses. The relative phase of one or more of the alternating electrical fields may be controlled to shape the pulses. For example the electrical fields may be at least one of:(a) in anti-phase at the start of each pulse; (b) in phase at the middle of each pulse; and (c) in anti-phase at the end of each pulse. The alternating electric fields may be controlled is that they are in anti-phase during the first intervals (e.g. between pulses).

The above and other embodiments and aspects of the disclosure may be employed in a method of providing a pulsed inferential signal for stimulation of a biological tissue, the method comprising frequency modulating at least one of a plurality of electric fields applied to the tissue while holding the amplitude (e.g. the amplitude of oscillation) of the electric fields constant. The frequency modulation of the at least one field is chosen to provide amplitude modulation of the combined field e.g. resulting from a superposition of the plurality of electric fields in the biological tissue. This can provide a series of pulses, and the duty cycle and/or pulse duration and/or interpulse interval of this series of pulses may be chosen according to the type of electrically excitable cells present in the tissue

An embodiment of the disclosure provides an electrical signal controller for controlling electro-stimulation of biological tissue, the controller being operable to control at least two alternating electrical signals and being configured to perform any one or more of the methods described and/or claimed herein. The frequency and/or duration of the pulses may be chosen according to the natural frequency band of the type of electrically excitable cells in the tissue. For example, the natural band may comprise a range of frequencies capable of evoking action potentials in the given type of electrically excitable cells. Such action potentials may be time locked with the sequence of pulses. For example, if the excitable cells comprise neurons, the modulated neural activity may comprise a neural spike train or neural oscillation, which may be time locked with the pulses.

To say that action potentials in a given excitable cell are "time-locked" with a sequence of peaks in an electrical waveform means that, for each respective peak in a sequence of peaks, an action potential occurs in the given cell after the respective peak and before the next peak if any in the sequence.

It will be appreciated from the discussion above that the embodiments shown in the Figures are merely exemplary, and include features which may be generalised, removed or replaced as described herein and as set out in the claims.

With reference to the drawings in general, it will be appreciated that schematic functional block diagrams are used to indicate functionality of systems and apparatus described herein. It will be appreciated however that the functionality need not be divided in this way, and should not be taken to imply any particular structure of hardware other than that described and claimed below. The function of one or more of the elements shown in the drawings may be further subdivided, and/or distributed throughout apparatus of the disclosure. In some embodiments the function of one or more elements shown in the drawings may be integrated into a single functional unit.

In some examples the control/processing functionality described herein may be provided by a general purpose processor or any other suitable controller, which may be configured to perform a method according to any one of those described herein. In some examples a suitable controller may comprise digital logic, such as field programmable gate arrays, FPGA, application specific integrated circuits, ASIC, a digital signal processor, DSP, or by any other appropriate hardware. In some examples, one or more memory elements can store data and/or program instructions used to implement the operations described herein. Embodiments of the disclosure provide tangible, non-transitory storage media comprising program instructions operable to program a processor to perform any one or more of the methods described and/or claimed herein and/or to provide data processing apparatus as described and/or claimed herein. The controller may comprise an analogue control circuit which provides at least a part of this control functionality. An embodiment provides an analogue control circuit configured to perform any one or more of the methods described herein.

Interferential stimulation of the present disclosure is not limited to stimulation of the brain, but has practical advantages in a wide variety of use cases. For example, interferential stimulation may be precisely targeted at any deep or superficial region of the body. For example, in some use scenarios, interferential stimulation may be targeted at particular regions of the heart, or at the pineal gland deep inside the cranium, or at the spinal cord or other nerves, or at the digestive tract, or at reproductive tissue, or at a muscle.

The present disclosure is not limited to interferential stimulation. Among other things, current isolation using an anti-phasic current channel may be employed to simultaneously deliver stimulation at different frequencies to different tissue regions, in such a manner that the tissue responds to the original waveforms, and not to the AM waveform created by interference.

Embodiments of the present disclosure may be employed in Temporal Interference (TI) stimulation of brain tissue, such as the human brain. This is a non-invasive brain stimulation method that uses first and second high frequency electrical fields. The first and second fields have frequencies of, $f_1$ and $f_2 = f_1 + \Delta f$, respectively.

The two frequencies differ by a small amount $\Delta f$ (e.g. $\Delta f$ is orders of magnitude smaller than $f_1$ & $f_2$ such that, $\Delta f$ « $f_1, f_2$) so that when the two fields interfere, they create a signal that is amplitude modulated (AM) at $\Delta f$. A stimulation locus corresponding to a high amplitude region of the AM TI signal is produced. The spatial position of the stimulation locus may be shifted by changing positioning of the electrodes.

Neuronal cells at the stimulation locus are stimulated with such an AM TI signal but not with the first and second high frequency fields alone. This means that stimulation can also be achieved in deep brain layers without activating overlying tissue.

For the avoidance of doubt, the disclosure of this application is intended to be considered as a whole. Any feature of any one of the examples disclosed herein may be combined with any selected features of any of the other examples described herein.

**Figures**

Some embodiments will now be described, by way of example only, with reference to the figures, in which:

> Figure 1 shows a schematic view of an apparatus for providing electrostimulation of a biological tissue;

> Figure 2 shows pulsed temporal interference waveforms produced by the apparatus of Figure 1;

> Figure 3 shows a shared time axis which compares the amplitude of pulsed temporal interference waveforms applied to neurons, neuron activations and spikes per second of neurons;

> Figure 4 shows a theoretical model of modified Hodgkin-Huxley (HH) cell response to pulsed TI stimulation of different frequencies;

> Figure 5 shows experimental data obtained from a non-focal motor response to a single electrode stimulation;

In the drawings like reference numerals are used to indicate like elements.

**Specific Description**

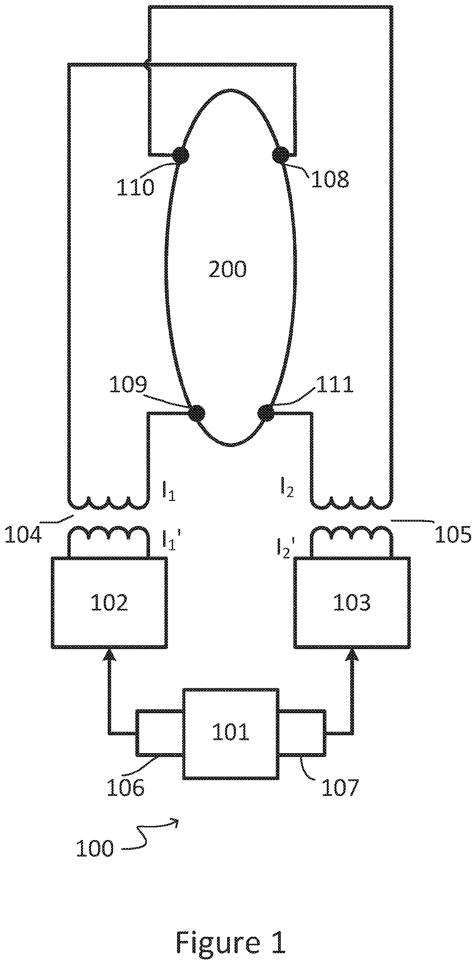

Figure 1 shows a schematic view of an apparatus 100 for providing electrostimulation of a biological tissue, the apparatus having, a controller 101 a first electrical signal provider 106 and a second electrical signal provider 107. As illustrated in Figure 1, the apparatus may be coupled to a biological tissue 200 so that the electrical signal providers can be used for applying two alternating electric fields to that tissue. Thus a first alternating electric field and a second alternating electric field may overlap in the biological tissue to provide a combined field.

The controller 101 is connected to the first electrical signal provider 106 and to the second electrical signal provider 107 and configured to cause variations in the frequency of the electrical signal produced by at least one of the two signal providers 106, 107. The controller is thereby able to frequency modulate one or both of the alternating electric fields to provide pulses (amplitude modulation) of the combined field in the tissue 200. This can provide a pulsed interferential stimulation signal, and the amplitude of each of the two electric fields can be held constant thereby reducing direct stimulation effects which might otherwise be caused by amplitude modulating either of the two electric fields themselves.

The first electrical signal provider 106 may be connected by a first current source 102, and a first transformer 104, to a first pair of electrodes 108, 109. This arrangement can be used to mediate the electric field due to the first electrical signal to the tissue 200. The second electrical signal provider 107 may be connected by a second current source 103, and a second transformer 105, to a second pair of electrodes 110, 111. This arrangement can be used to mediate the electric field due to the second electrical signal to the tissue 200. The current sources 102, 103, may each comprise a voltage controlled current source connected to be controlled by a voltage output provided by the controller 101.

The controller 101 may comprise a data generator configured to provide digital data representing samples of an alternating signal (e.g. a waveform) such as a sinusoid. The controller 101 can thus prepare a first waveform and a second waveform and control the first signal provider and the second signal provider to provide output voltages representing each of these waveforms e.g. It will be appreciated that any signal generator can be used to provide these output voltages - one example of such a signal provider is an output channel of a DAC (digital to analogue converter). The controller may thus control the signal providers to provide the first waveform in the form of a first voltage $V_1$ to the first current source 102, and the second waveform in the form of a second voltage $V_2$ to the second current source 103.

The first current source 102 can thus be configured to provide a first current source (CS) current $I_1'$ based on the voltage $V_1$ received from the controller 101. This current may comprise an alternating current, such as a sinusoid. The first CS current $I_1'$ is passed through a primary coil of the first transformer 104 to induce an alternating first current $I_1$ in the secondary coil of that transformer 104. This alternating first current $I_1$ may thus be isolated from DC offset voltages in the current source so that it can be applied to a first pair of electrodes 108, 109 for causing a first electric field in the biological tissue. The second coil of the first transformer may be grounded e.g. connected to a reference voltage. The first transformer may have a turns ratio of 1, in which case, the first CS current is equal to the first current ($I_1' = I_1$).

The second current source 103 may similarly be configured to provide a second current source (CS) current $I_2$' based on the voltage $V_2$ received from the controller 101. This can be connected to a second transformer 105 arranged identically to the first transformer 104. A second current $I_2$ can thus be provided which is isolated from DC offset voltages in the current source so that it can be applied to a second pair of electrodes 110, 111 for causing a second electric field in the biological tissue. The secondary coil of the second transformer may also be grounded e.g. by being connected to the same reference voltages as the first transformer. The second transformer may have a turns ratio of 1, in which case the second CS current is equal to the second current ($I_2$' = $I_2$).

The alternating first and second currents, $I_1$ and $I_2$ respectively, may be sinusoidal. The first and second currents may be described mathematically as follows:

$$I_1 = A_1 \cos(2\pi f_1 t + \varphi_1)$$

$$I_2 = A_2 \cos(2\pi f_2 t + \varphi_2)$$

Wherein A is the amplitude, f is the frequency, $\varphi$ is the phase, t is the time and the subscripts 1 and 2 denote which current signal the respective parameters describe.

In operation, the electrodes 108, 109, 110, 111 may be coupled to the biological tissue to provide a first alternating electric field between the first electrodes 108 and 109, and a second alternating electric field between the second electrodes 110, 111. For example they may be located around the biological tissue such that a straight line between the first pair of electrodes 108, 109 intersects at least part of the biological tissue 200. The second electrodes 110, 111 may also be located such that a straight line between the second pair of electrodes intersects at least part of the biological tissue 200, including a part between the first pair of electrodes 108, 109. This part, e.g. a target region of the biological tissue, may thus lie between both pairs of electrodes so that the electric fields from the two pairs of electrodes can combine in the target region of the biological tissue.

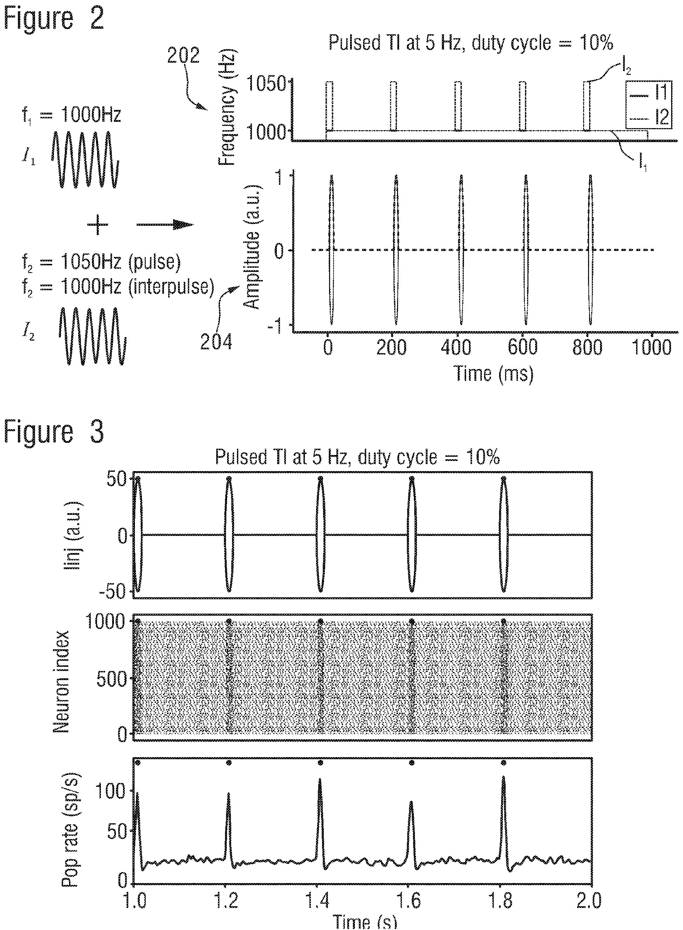

Operation of this arrangement in a method for providing pulses of interferential stimulation by electric field will now be described with reference to Figure 2.

Figure 2 comprises a first plot 202 in which the vertical axis indicates frequency, and the horizontal axis indicates the passage of time. This first plot includes a first time series indicating the frequency of the first alternating electrical signal $I_1$ (e.g. provided at the first electrodes 108, 109), and a second time series indicating the frequency of the second alternating electrical signal $I_2$ (e.g. provided at the first electrodes 108, 109).

Figure 2 also comprises a second plot 204 in which the vertical axis indicates electric field (e.g. the instantaneous electric field value) in arbitrary units. This second plot shows a single time series indicating the combined field produced in the target tissue. This single time series is however illustrated in terms of the instantaneous scalar value of the oscillating combined electric field, and the envelope of the amplitude modulation of that oscillating (alternating) electric field is also shown for the purposes of illustration.

The two plots share a common time axis, which is gradated in milliseconds over an interval of one second.

The frequency of the first alternating electrical signal $I_1$ is held constant by the controller throughout the illustrated interval. This is indicated by a straight horizontal line on the first plot indicating a constant frequency maintained throughout. This frequency may be much higher than the natural band of the electrically excitable cells in the tissue. For example, it may be 1000Hz as shown in the first plot 202 of Figure 2.

The frequency of the second alternating electrical signal $I_2$ may be controlled, by the controller 101, as a boxcar wave (e.g. a sequence of square pulses). As illustrated in the first plot of Figure 2, the pulses of this boxcar wave in $I_2$ begin at t=0ms. The frequency of $I_2$ at this instant may match (e.g. be equal to) the frequency of the first alternating electrical signal, e.g. 1000Hz in Figure 2. The controller 101 then changes, e.g. raises, The frequency of the second alternating electrical signal by a frequency difference $\Delta f$ (e.g. 50 Hz) to a second frequency for the duration of the pulse (e.g. 20ms), before returning to the frequency of the first alternating electrical signal, e.g. 1000Hz in Figure 2. The pulses in frequency of the second alternating signal then repeat with a period selected according to the tissue and/or excitable cell type which is to be stimulated - in Figure 2 this is shown as 200 ms.

The second plot shown in Figure 2 illustrates the instantaneous amplitude of the combined field provided by the above described frequency modulation of the two alternating electric fields. The plot shows the amplitude modulated envelope of the combined field, superposed on the field itself. Although not clearly visible in the second plot of Figure 2, during the intervals in which the two alternating signals differ by the frequency difference, the combined field oscillates with a frequency which is equal to the average of the first frequency and the second frequency, e.g. 1025 Hz. The envelope of that oscillation however corresponds to the beat frequency (e.g. the difference in frequency between the two applied electric fields). This is of course manifest as a measurable frequency component of the oscillation of the combined field. It can thus be seen that, by control of the frequency of the two applied electric fields, pulses of electric field in the natural band of the electrically excitable cells can be provided without the need to amplitude modulate either of the two signals.

The controller 101 may be configured to provide a phase shift, $\varphi_1$, $\varphi_2$ to either or both of the two current signals, and to set these phase shifts so that the two alternating electrical signals are in antiphase at the start of each pulse of the combined field. The phase shift may also be controlled so that the two alternating electrical signals are in phase at the middle of the pulse and/or in antiphase again at the end of each pulse of the combined field. The provision of this controlled phase shift may provide improved pulse shaping, which may avoid energy leaking into undesired frequency bands e.g. outside the natural band of the electrically excitable cells.

The controller 101 is configured to provide variations of at least one of the first frequency $f_1$ and the second frequency $f_2$ so that the combined field provides a pulsed interferential stimulation signal. The variations provide a frequency difference, $\Delta f$, selected to provide stimulation of the biological tissue. For example, the frequency of the first alternating electric field may be varied by $\pm\Delta f/2$ and the frequency of the second alternating electric field may be varied by $+\Delta f/2$. For example, the first frequency and the second frequency are both greater than a minimum frequency selected to avoid stimulation of the biological tissue. For example, when the biological tissue comprises brain or spinal cord tissue the minimum frequency may be at least 500Hz, for example at least 800 Hz, for example at least 1KHz to prevent stimulation of the biological tissue.

Optionally the apparatus may be configured to hold constant the amplitude of the first alternating electric field and the second alternating electric field. The term constant is understood to mean sufficiently constant over time periods equal to, or greater than, the pulse time period (e.g. the inverse of the pulse frequency). This allows the first and second alternating electric fields to destructively interfere to produce a combined field with zero amplitude at regions of the biological tissue wherein stimulation is not desired.

The pulses may be configured to have a duration of at least 5ms, or have a duration of less than 150ms (for example less than 25ms), or have a duration of between 5 ms and 25 ms. The values for the durations herein refer to the complete pulse width.

In use, the first waveform and the second waveform are prepared by the controller 101. The controller provides the first voltage $V_1$ based on the first waveform, to the first current source 102. The controller provides the second voltage $V_2$ based on the second waveform, to the second current source 103. The first current source provides the first current source (CS) current $I_1'$ based on the first output voltage $V_1$. The first CS current $I_1'$ is passed through the first transformer 104 to induce the first current $I_1$, e.g. between the electrodes 108, 109. Similarly, the second current source provides the second current source (CS) current $I_2'$ based on the second output voltage $V_2$. The second CS current $I_2'$ is passed through the second transformer 105 to induce the second current $I_2$, e.g. between the electrodes 110, 111. The first electrical signal provider can thus provide the first alternating electric field in the biological tissue 200, the first alternating electric field having the first frequency $f_1$. Likewise,

the second electrical signal provider can thus provide the second alternating electric field in the biological tissue 200, the second alternating electric field having the second frequency $f_2$.

As discussed above, the first and second alternating electric fields may be proportional to the first and second alternating currents - e.g. depending on the electrical impedance between the respective pairs of electrodes.

Figure 3 shows three plots having a common time axis. These plots show the amplitude of pulsed temporal interference waveforms applied to neurons, neuron activations and spikes per second of neurons. The plots were obtained using neural network modelling.

The first plot indicates the combined field applied to a biological tissue, and shows a series of pulses such as those described above with reference to the second plot of Figure 2. In Figure 3, the pulses in the combined field have a 20ms duration, and a 200ms period (e.g. a 10% duty cycle).

The second plot indicates excitation of excitable cells in the biological tissue. The third plot indicates the total number of excited cells as a function of time.

These results show that during times where a pulse is provided with a frequency within the cells' natural band and with an amplitude exceeding a threshold amplitude, the number of neurons stimulated increases, therefore demonstrating the ability of the apparatus to stimulate neuron activity (neuron spikes).

The size, shape and position of a region where the envelope amplitude exceeds a given threshold depends on the relative amplitudes of the two current channels and on the placement of the electrodes for the two channels. In some aspects of the disclosure, these factors are adjusted to precisely position this region at the targeted tissue. For example, in some scenarios involving interferential stimulation, transcranial electrodes create electric fields in a brain such that: (i) an interferential zone is created close to one or more of the electrodes at a superficial depth in the brain (e.g. in the cortex); (ii) an interferential zone is created at a deeper depth of the brain but laterally close to the electrodes; or (iii) an interferential zone is created at any brain depth in a region that it is remote from the electrodes.

Optionally the first electrode pair and second electrode pair may be positioned such that, at a given time, the largest magnitude of the envelope amplitude occurs in only one region of the brain. This region is path-connected and consists only of those points at which the largest magnitude of the envelope amplitude occurs. This region of highest envelope amplitude may be precisely targeted. For example, in some aspects, this region of highest envelope amplitude is positioned such that the region spatially coincides with (i) cortical tissue of a brain, (ii) subcortical tissue of a brain; (iii) heart tissue, or (iv) tissue in a nerve. More generally, this region of highest envelope amplitude may be precisely positioned on target tissue anywhere in the body.

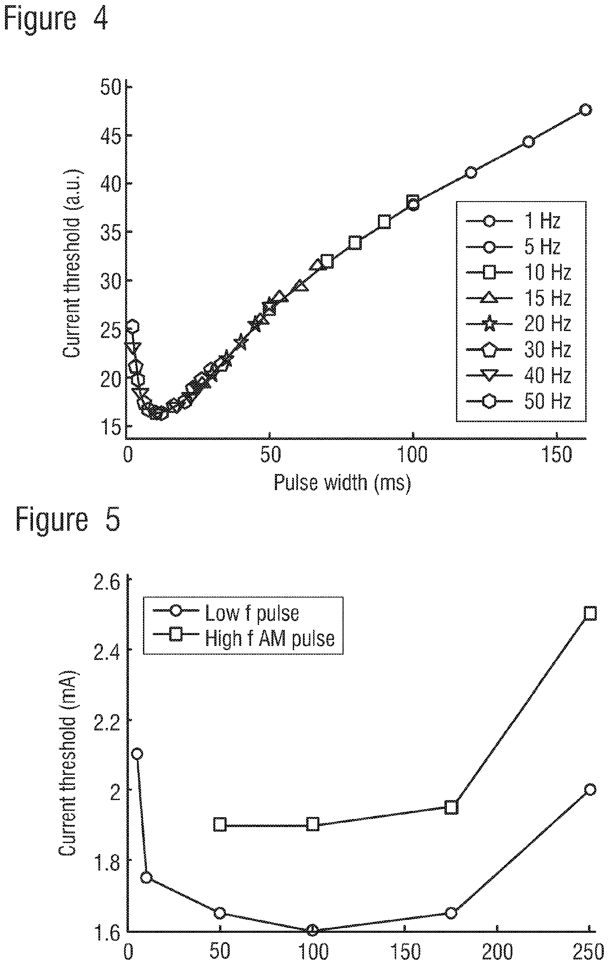

Figure 4 shows a theoretical model of modified Hodgkin-Huxley (HH) cell response to pulsed TI stimulation of different frequencies at varying duty cycles (10-100% range in increments of 10%). Thresholds for pulse widths > ~150 ms could not be found with the HH model, also when classical model was tested with conventional bipolar pulses.

Response of a modified Hodgkin-Huxley (HH) cell to pTI current injections was modelled. In this model. It was predicted that the cell responds preferentially to pulses in a clear optimum pulse width region (~5-25 ms).

Figure 5 shows experimental data obtained from a non-focal motor response to a single electrode stimulation shows preferential response to pulse widths of 50-100 ms (n=1).

A non-focal motor response was evoked using a single electrode pair positioned on a mouse brain (coordinates relative to bregma: stimulating electrode on AP -1.5 mm, ML +0.5 mm, sink on AP 1.5mm, ML +0.5mm), with either low frequency (4 Hz) or high frequency amplitude modulated (4 Hz at a carrier frequency of 1000 HZ) pulses of varying pulse duration. Both stimulation types show similar modulation of current threshold by pulse width and decrease of threshold by ~20-25% in 50-100 ms range. Motor responses evoked consisted of paw, whisker and hindpaw movement.

The embodiments described above are intended merely to be exemplary not to be limiting. For example, the arrangement of electrical connectors between the signal providers and the tissue may be different - the transformers are optional, and may be replaced by any appropriate connection system. Voltage controlled current sources and grounding are mentioned as an option but other ways of providing the electric fields and stimulating the tissue may be used. The electrodes 108, 109, 110, 111 are described as being coupled to the biological tissue. This coupling may comprise a direct electrical connection, for example the electrodes may be directly connected to the biological tissue, e.g. by being implanted in it. The coupling however may also be indirect, and may be mediated by other biological and/or non-biological structures. For example, the electrodes may be placed on the skin (e.g. the scalp) of a subject for targeting excitable cells in a target region of tissue (such as brain tissue) which is disposed between the locations to which the electrodes are affixed. One example of the arrangement of electrodes is shown in Figure 1, but the electrode locations may be chosen differently according to need.

As another example - it is explained above that one of the two frequencies may be held constant while the other is modulated, but this is optional. For example, rather than modulating one frequency by $\Delta f$, it is possible instead to modulate both frequencies but each in the opposite sense, so that the frequency of one signal is reduced while the other is increased, for example one may be increased (e.g. by $\Delta f/x$) while the other is reduced by a corresponding amount (e.g. by $\Delta f(1-1/x)$). The two changes in frequency may be equal and opposite, but any combination of changes may be chosen to chosen to provide the desired frequency difference of $\Delta f$ for the duration of the pulse.

Digital signal generation has been described (e.g. using digital samples of a waveform) and this has some advantages, for example because pre-generated waveforms can simply be played out and/or because it can allow precise control of instantaneous phase. However, analogue signal generators such as tuneable tank circuits can also be used.

The electrically excitable cells described herein may comprise any one or more types of cells selected from the list comprising neurons, muscle cells (skeletal, cardiac, and smooth), and some endocrine cells (e.g., insulin-releasing pancreatic β cells).

A neuronal response to an electrical stimulus can be affected by its duration and pattern. For example, long pulse stimuli can increase neuronal spiking threshold and specific pulse successions such as theta burst stimulation can more effectively induce long-lasting plasticity effects, also in humans. Temporal Interference (TI) is a non-invasive deep brain stimulation technique that uses high frequency alternating currents that stimulate neurons regularly at their frequency difference. While TI is capable of stimulating deep layers without overlying cortex, its strength drops with the stimulation depth.

It will thus be appreciated, having read the preceding disclosure, that a TI apparatus for providing pulsed electrostimulation of a biological tissue is provided. The apparatus generally comprises: a first electrical signal provider for providing a first alternating electric field in the biological tissue, the first alternating electric field having a first frequency; a second electrical signal provider for providing a second alternating electric field in the biological tissue the second alternating electric field having a second frequency; wherein the first alternating electric field and the second alternating electric field provide a combined field in the biological tissue, and the apparatus comprises a controller configured to provide variations of at least one of the first frequency and the second frequency so that the combined field provides a pulsed interferential stimulation signal. This apparatus finds a number of useful applications such as those described herein and further applications and uses of this apparatus will be apparent to the skilled addressee in the context of the present disclosure.

**Claims**

1. An apparatus for providing electrostimulation of a biological tissue, the apparatus comprising:

   a first electrical signal provider (106) for providing a first alternating electric field in the biological tissue, the first alternating electric field having a first frequency;
   a second electrical signal provider (107) for providing a second alternating electric field in the biological tissue the second alternating electric field having a second frequency;

wherein the first alternating electric field and the second alternating electric field provide a combined field in the biological tissue, and the apparatus comprises a controller (101) configured to provide variations of at least one of the first frequency and the second frequency so that the combined field provides a pulsed interferential stimulation signal, wherein the variations provide a series of first intervals during which the first frequency matches the second frequency, and the first intervals are interleaved with a series of second intervals during which the first frequency is different from the second frequency.

2. The apparatus of claim 1 wherein the apparatus is configured to hold constant the amplitude of the first alternating electric field and the second alternating electric field.

3. The apparatus of claims 1 or 2 in which the frequency difference during the second intervals provides amplitude modulation of the interferential stimulation signal having a beat period, and the second intervals have a duration which is an integer multiple of the beat period.

4. The apparatus of any preceding claim wherein the variations provide a frequency difference, Δf, selected to provide stimulation of the biological tissue, e.g. by excitation in the natural band of electrically excitable cells in the biological tissue.

5. The apparatus of any preceding claim wherein the first frequency and the second frequency are both selected to avoid stimulation of the biological tissue, for example both are outside the natural band of electrically excitable cells in the biological tissue, for example both above a minimum frequency which is outside the natural band.

6. The apparatus of claim 5, wherein the biological tissue comprises brain or spinal tissue and the minimum frequency is at least 500Hz, for example at least 800 Hz, for example at least 1KHz.

7. The apparatus of any preceding claim wherein

the first electrical signal provider comprises a first current source for providing electrical current between a first two electrodes, thereby to provide the first electric field, and
the second electrical signal provider comprises a second current source for providing electrical current between two electrodes, thereby to provide the second electric field.

8. The apparatus of claim 7 wherein the electrodes comprise electrodes for implantation into a living biological tissue, such as brain tissue and/or for being secured near the tissue - e.g. on the scalp.

9. The apparatus of any of claims 1 to 8 wherein the pulses have a duration of at least 5ms.

10. The apparatus of any preceding claim in which the pulses have a duration of less than 150ms, for example less than 25ms.

11. The apparatus of claim 10 wherein the pulses have a duration of between 5 ms and 25 ms.

12. A method of stimulating biological tissue the method comprising:

controlling a first alternating current at a first location thereby to provide a first alternating electric field in the biological tissue, the first alternating electric field having a first frequency;
providing a second alternating current at a second location thereby to provide a second alternating electric field in the biological tissue the second alternating electric field having a second frequency;
wherein the first electric field and the second electric field combine to provide a combined field in the biological tissue at a third location, and the method comprises:

varying at least one of the first frequency and the second frequency so that the combined field provides a pulsed interferential stimulation signal;
providing a series of first intervals during which the first frequency matches the second frequency; and
providing a series of second intervals interleaved with the first intervals during which the frequency difference between the two electric fields provides amplitude modulation of the combined field
wherein the biological tissue is *ex vivo* and/or wherein the method is not a method of treatment of the living human or animal body by surgery or therapy.

13. The method of claim 12 comprising keeping the amplitude of the first alternating electric field and the second alternating electric field constant during said varying.

14. The method of claim 12 or 13 wherein varying at least one of the first frequency and the second frequency comprises varying the first frequency while keeping the second frequency constant.

15. The method of any of claims 12 to 14 wherein at least one of the first location and the second location is in the biological tissue.

**Patentansprüche**

1. Eine Vorrichtung zur Bereitstellung von Elektrostimulation eines biologischen Gewebes, wobei die Vorrichtung Folgendes umfasst:

   einen ersten elektrischen Signalgeber (106) zur Bereitstellung eines ersten elektrischen Wechselfelds in dem biologischen Gewebe, wobei das erste elektrische Wechselfeld eine erste Frequenz aufweist;
   einen zweiten elektrischen Signalgeber (107) zur Bereitstellung eines zweiten elektrischen Wechselfelds in dem biologischen Gewebe, wobei das zweite elektrische Wechselfeld eine zweite Frequenz aufweist;
   wobei das erste elektrische Wechselfeld und das zweite elektrische Wechselfeld ein kombiniertes Feld in dem biologischen Gewebe bereitstellen und die Vorrichtung eine Steuerung (101) umfasst, die dazu ausgelegt ist, Variationen von mindestens einem von der ersten Frequenz und der zweiten Frequenz bereitzustellen, sodass das kombinierte Feld ein gepulstes Interferenzstimulationssignal bereitstellt, wobei die Variationen eine Reihe von ersten Intervallen bereitstellen, während derer die erste Frequenz mit der zweiten Frequenz übereinstimmt, und die ersten Intervalle mit einer Reihe von zweiten Intervallen verschachtelt sind, während derer die erste Frequenz von der zweiten Frequenz verschieden ist.

2. Vorrichtung nach Anspruch 1, wobei die Vorrichtung dazu ausgelegt ist, die Amplitude des ersten elektrischen Wechselfelds und des zweiten elektrischen Wechselfelds konstant zu halten.

3. Vorrichtung nach Ansprüchen 1 oder 2, wobei der Frequenzunterschied während der zweiten Intervalle eine Amplitudenmodulation des Interferenzstimulationssignals mit einer Schwebungsdauer bereitstellt und die zweiten Intervalle eine Dauer aufweisen, die ein ganzzahliges Mehrfaches der Schwebungsdauer ist.

4. Vorrichtung nach einem der vorhergehenden Ansprüche, wobei die Variationen einen Frequenzunterschied Δf bereitstellen, der ausgewählt ist, um eine Stimulation des biologischen Gewebes bereitzustellen, z. B. durch Erregung im natürlichen Band elektrisch erregbarer Zellen in dem biologischen Gewebe.

5. Vorrichtung nach einem der vorhergehenden Ansprüche, wobei die erste Frequenz und die zweite Frequenz beide ausgewählt sind, um eine Stimulation des biologischen Gewebes zu vermeiden, wobei zum Beispiel beide außerhalb des natürlichen Bands elektrisch erregbarer Zellen in dem biologischen Gewebe liegen, zum Beispiel beide über einer außerhalb des natürlichen Bands liegenden Mindestfrequenz liegen.

6. Vorrichtung nach Anspruch 5, wobei das biologische Gewebe Gehirn- oder Rückenmarkgewebe umfasst und die Mindestfrequenz mindestens 500 Hz, zum Beispiel mindestens 800 Hz, zum Beispiel mindestens 1 KHz, beträgt.

7. Vorrichtung nach einem der vorhergehenden Ansprüche, wobei

   der erste elektrische Signalgeber eine erste Stromquelle zur Bereitstellung von elektrischem Strom zwischen ersten zwei Elektroden umfasst, um somit das erste elektrische Feld bereitzustellen, und
   der zweite elektrische Signalgeber eine zweite Stromquelle zur Bereitstellung von elektrischem Strom zwischen zwei Elektroden umfasst, um somit das zweite elektrische Feld bereitzustellen.

8. Vorrichtung nach Anspruch 7, wobei die Elektroden Elektroden zur Implantation in ein lebendes biologisches Gewebe, wie etwa Gehirngewebe, und/oder zur Befestigung in der Nähe des Gewebes - z. B. auf der Kopfhaut, umfassen.

9. Vorrichtung nach einem der Ansprüche 1 bis 8, wobei die Impulse eine Dauer von mindestens 5 ms aufweisen.

10. Vorrichtung nach einem der vorhergehenden Ansprüche, wobei die Impulse eine Dauer von weniger als 150 ms, zum Beispiel weniger als 25 ms, aufweisen.

11. Vorrichtung nach Anspruch 10, wobei die Impulse eine Dauer zwischen 5 ms und 25 ms aufweisen.

12. Ein Verfahren zum Stimulieren von biologischem Gewebe, wobei das Verfahren Folgendes umfasst:

   Steuern eines ersten Wechselstroms an einem ersten Ort, um somit ein erstes elektrisches Wechselfeld in dem biologischen Gewebe bereitzustellen, wobei das erste elektrische Wechselfeld eine erste Frequenz aufweist;
   Bereitstellen eines zweiten Wechselstroms an einem zweiten Ort, um somit ein zweites elektrisches Wechselfeld in dem biologischen Gewebe bereitzustellen, wobei das zweite elektrische Wechselfeld eine zweite Frequenz aufweist;

wobei das erste elektrische Feld und das zweite elektrische Feld sich kombinieren, um an einem dritten Ort ein kombiniertes Feld in dem biologischen Gewebe bereitzustellen, und wobei das Verfahren Folgendes umfasst:

Variieren mindestens einer von der ersten Frequenz und der zweiten Frequenz, sodass das kombinierte Feld ein gepulstes Interferenzstimulationssignal bereitstellt;

Bereitstellen einer Reihe von ersten Intervallen, während derer die erste Frequenz mit der zweiten Frequenz übereinstimmt; und

Bereitstellen einer Reihe von zweiten Intervallen, die mit den ersten Intervallen verschachtelt sind, während derer der Frequenzunterschied zwischen den zwei elektrischen Feldern eine Amplitudenmodulation des kombinierten Felds bereitstellt, wobei das biologische Gewebe ex vivo ist und/oder wobei das Verfahren kein Verfahren zum Behandeln des lebenden menschlichen oder tierischen Körpers durch Chirurgie oder Therapie ist.

**13.** Verfahren nach Anspruch 12, das die Konstanthaltung der Amplitude des ersten elektrischen Wechselfelds und des zweiten elektrischen Wechselfelds während des genannten Variierens umfasst.

**14.** Verfahren nach Anspruch 12 oder 13, wobei das Variieren mindestens einer von der ersten Frequenz und der zweiten Frequenz das Variieren der ersten Frequenz, während die zweite Frequenz konstant gehalten wird, umfasst.

**15.** Verfahren nach einem der Ansprüche 12 bis 14, wobei mindestens einer von dem ersten Ort und dem zweiten Ort in dem biologischen Gewebe liegt.

## Revendications

**1.** Appareil conçu pour produire une électrostimulation d'un tissu biologique, l'appareil comprenant :

un premier élément de fourniture de signal électrique (106) conçu pour produire un premier champ électrique alternatif dans le tissu biologique, le premier champ électrique alternatif ayant une première fréquence ;

un deuxième élément de fourniture de signal électrique (107) conçu pour produire un deuxième champ électrique alternatif dans le tissu biologique, le deuxième champ électrique alternatif ayant une deuxième fréquence ;

dans lequel le premier champ électrique alternatif et le deuxième champ électrique alternatif produisent un champ combiné dans le tissu biologique, et l'appareil comprend un dispositif de commande (101) configuré pour produire des variations d'au moins l'une de la première fréquence et de la deuxième fréquence de telle sorte que le champ combiné produise un signal pulsé de stimulation interférentielle, dans lequel les variations produisent une série de premiers intervalles durant lesquels la première fréquence correspond à la deuxième fréquence, et les premiers intervalles sont intercalés avec une série de deuxièmes intervalles durant lesquels la première fréquence est différente de la deuxième fréquence.

**2.** Appareil selon la revendication 1, l'appareil étant configuré pour maintenir constante l'amplitude du premier champ électrique alternatif et du deuxième champ électrique alternatif.

**3.** Appareil selon les revendications 1 ou 2, dans lequel la différence de fréquence durant les deuxièmes intervalles produit une modulation d'amplitude du signal de stimulation interférentielle ayant une période de battement, et les deuxièmes intervalles ont une durée qui est un multiple entier de la période de battement.

**4.** Appareil selon l'une quelconque des revendications précédentes, dans lequel les variations produisent une différence de fréquence, Δf, sélectionnée pour produire une stimulation du tissu biologique, par exemple par excitation dans la bande naturelle de cellules électriquement excitables dans le tissu biologique.

**5.** Appareil selon l'une quelconque des revendications précédentes, dans lequel la première fréquence et la deuxième fréquence sont toutes deux sélectionnées pour éviter une stimulation du tissu biologique, par exemple les deux sont en dehors de la bande naturelle de cellules électriquement excitables dans le tissu biologique, par exemple les deux sont au-dessus d'une fréquence minimale qui se trouve en dehors de la bande naturelle.

**6.** Appareil selon la revendication 5, le tissu biologique comprenant un tissu cérébral ou rachidien et la fréquence minimale étant d'au moins 500 Hz, par exemple d'au moins 800 Hz, par exemple d'au moins 1 KHz.

**7.** Appareil selon l'une quelconque des revendications précédentes, dans lequel

le premier élément de fourniture de signal électrique comprend une première source de courant pour produire un courant électrique entre deux premières électrodes, en produisant ainsi le premier champ électrique, et
le deuxième élément de fourniture de signal électrique comprend une deuxième source de courant pour produire un courant électrique entre deux électrodes, en produisant ainsi le deuxième champ électrique.

8. Appareil selon la revendication 7, dans lequel les électrodes comprennent des électrodes destinées à être implantées dans un tissu biologique vivant, tel qu'un tissu cérébral, et/ou à être fixées près du tissu - par exemple sur le cuir chevelu.

9. Appareil selon l'une quelconque des revendications 1 à 8, dans lequel les impulsions ont une durée d'au moins 5 ms.

10. Appareil selon l'une quelconque des revendications précédentes, dans lequel les impulsions ont une durée inférieure à 150 ms, par exemple inférieure à 25 ms.

11. Appareil selon la revendication 10, dans lequel les impulsions ont une durée comprise entre 5 ms et 25 ms.

12. Procédé de stimulation d'un tissu biologique, le procédé comprenant :

la régulation d'un premier courant alternatif à un premier emplacement, en produisant ainsi un premier champ électrique alternatif dans le tissu biologique, le premier champ électrique alternatif ayant une première fréquence ;
la production d'un deuxième courant alternatif à un deuxième emplacement, en produisant ainsi un deuxième champ électrique alternatif dans le tissu biologique, le deuxième champ électrique alternatif ayant une deuxième fréquence ;
dans lequel le premier champ électrique et le deuxième champ électrique se combinent pour produire un champ combiné dans le tissu biologique à un troisième emplacement, et le procédé comprend :

le fait de faire varier au moins l'une de la première fréquence et de la deuxième fréquence de telle sorte que le champ combiné produise un signal pulsé de stimulation interférentielle ;
la production d'une série de premiers intervalles durant lesquels la première fréquence correspond à la deuxième fréquence ; et
la production d'une série de deuxièmes intervalles intercalés avec les premiers intervalles durant lesquels la différence de fréquence entre les deux champs électriques produit une modulation d'amplitude du champ combiné ;
dans lequel le tissu biologique est *ex vivo* et/ou dans lequel la méthode n'est pas une méthode de traitement du corps humain ou animal vivant par chirurgie ou thérapie.

13. Procédé selon la revendication 12, comprenant le fait de maintenir constante l'amplitude du premier champ électrique alternatif et du deuxième champ électrique alternatif durant ladite variation.

14. Procédé selon la revendication 12 ou 13, dans lequel la variation d'au moins l'une de la première fréquence et de la deuxième fréquence comprend le fait de faire varier la première fréquence tout en maintenant constante la deuxième fréquence.

15. Procédé selon l'une quelconque des revendications 12 à 14, dans lequel au moins l'un du premier emplacement et du deuxième emplacement se trouve dans le tissu biologique.

Figure 1

## Figure 2

$f_1 = 1000\text{Hz}$

$I_1$

$+$

$f_2 = 1050\text{Hz (pulse)}$
$f_2 = 1000\text{Hz (interpulse)}$

$I_2$

202

204

Pulsed TI at 5 Hz, duty cycle = 10%

## Figure 3

Pulsed TI at 5 Hz, duty cycle = 10%

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016057855 A
- US 5324317 A
- EP 0002811 A1